# EUROPEAN PATENT SPECIFICATION

(11) **EP 4 547 410 B1**
(45) Date of publication and mention of the grant of the patent: **20.05.2026**
(21) Application number: 23745719.7
(22) Date of filing: 28.06.2023
(51) Int. Cl.: B05B 7/14, A61M 13/00

(54) **ASSEMBLIES FOR DELIVERING AGENTS**
ANORDNUNGEN ZUR ABGABE VON WIRKSTOFFEN
ENSEMBLES D'ADMINISTRATION D'AGENTS

(30) Priority: 01.07.2022 US 202263357883 P
(43) Date of publication of application: 07.05.2025
(73) Proprietor: Boston Scientific Scimed Inc., Maple Grove, Minnesota 55311 (US)
(72) Inventor: WHELEHAN, Jennifer, Worcester, Massachusetts 01609 (US); NAM, Ra, Lawrence, Massachusetts 01843 (US); GOLDEN, John B., Norton, Massachusetts 02766-3507 (US); LIMBERG, Pauline Rosemary, Northborough, Massachusetts 01532 (US); PLEAU, Dayna M., South Attleboro, Massachusetts 02703 (US); PIC, Andrew, Northboro, Massachusetts 01532 (US)
(74) Representative: Vossius & Partner Patentanwälte Rechtsanwälte mbB
(86) International application number: PCT/US2023/069232
(87) International publication number: WO 2024/006807

(56) References cited:
- US-A1- 2019 232 030
- US-A1- 2021 162 122
- US-A1- 2021 275 157

## Description

### CROSS-REFERENCE TO RELATED APPLICATIONS

This application claims the benefit of priority of U.S. Provisional Patent Application No. 63/357,883, filed on July 1, 2022.

### TECHNICAL FIELD

Various aspects of this disclosure relate generally to devices, assemblies, and methods for delivering agents. More specifically, the disclosure relates to devices and assemblies for delivery of powdered agents, such as hemostatic agents. The invention is defined in independent claim 1. Preferred embodiments are matter of the appended dependent claims.

### BACKGROUND

In certain medical procedures, it may be necessary to minimize or stop bleeding internal to the body. For example, an endoscopic medical procedure may require hemostasis of bleeding tissue within the gastrointestinal tract, for example, in the esophagus, stomach, or intestines.

During an endoscopic procedure, a user inserts a sheath of an endoscope into a body lumen of a patient. The user utilizes a handle of the endoscope to control the endoscope during the procedure. The user may pass one or more through a working channel of the endoscope via, for example, a port in the handle, to deliver treatment at the procedure site near a distal end of the endoscope. The procedure site is remote from the operator.

To achieve hemostasis at the remote site, a hemostatic agent may be delivered by a device inserted into the working channel of the endoscope. Agent delivery may be achieved through systems that may be manually operated, for example. Such systems, however, may require numerous steps or actuations to achieve delivery, or may not achieve a desired rate of agent delivery or a desired dosage of agent. Further, the systems may result in the agent clogging portions of the delivery device, inconsistent dosing of agent, or may not result in the agent reaching the treatment site deep within the GI tract.

US 2021/0275157 A1 discloses a device for delivering an agent which may comprise a housing defining an enclosure. The housing may be configured to store an agent. The device may further comprise an inlet, in fluid communication with the enclosure, for receiving a flow of pressurized fluid; an outlet in fluid communication with the enclosure; and a filter disposed within the enclosure. Pores of a wall of the filter may be configured such that the fluid is permitted to pass through the pores into a channel defined by an inner surface of the wall. An actuation member may be configured to transition between a first configuration, in which portions of the agent disposed proximally to the actuation member are prevented from passing distally of the actuation member, and a second configuration, in which portions of the agent disposed proximally of the actuation member are capable of passing distally of the actuation member.

The current disclosure may solve one or more of these issues or other issues in the art.

### SUMMARY

Each of the aspects disclosed herein may include one or more of the features described in connection with any of the other disclosed aspects.

The claimed invention is directed at a valve assembly for a medical device. The valve assembly includes an enclosure for storing an agent, the enclosure including a first end; a funnel positioned downstream of the enclosure for receiving the agent from the enclosure, the funnel including a second end positioned adjacent to the first end; a first plate coupled to the enclosure at the first end, the first plate including at least one opening; and a second plate coupled to the funnel at the second end, the second plate including at least one opening that is in fluid communication with the at least one opening of the first plate. The first plate and the second plate are positioned against one another, and an exterior surface of at least one of the first plate or the second plate includes one or more textured surface elements configured to displace the agent stored in the enclosure in response to at least one of the first plate or the second plate moving relative to one another, wherein the one or more textured surface elements is configured to vibrate the enclosure, in response to at least one of the first plate or the second plate moving relative to one another, to displace the agent stored in the enclosure.

The claimed valve assembly may include any of the following features. The one or more textured surface elements includes a ridge, a protrusion, a projection, a bump, a rib, a tooth, a baffle, a dimple, a recess, or a cavity. The first plate is configured to rotate or translate relative to the second plate. The one or more textured surface elements is configured to agitate the agent in the enclosure and release the agitated agent from the enclosure and into the funnel in response to at least one of the first plate or the second plate moving relative to one another. The exterior surface of each of the first plate and the second plate include one or more textured surface elements. The one or more textured surface elements positioned on the exterior surface of the first plate are configured to engage the one or more textured surface elements positioned on the exterior surface of the second plate, thereby displacing the agent stored in the enclosure. At least one of the first plate or the second plate is configured to translate in a longitudinal direction relative to one another when the one or more textured surface elements of the first plate engage the one or more textured surface elements of the second plate. Each of the first plate and the second plate are configured to move relative to one another to displace the agent stored in the enclosure for release into the funnel. The one or more textured surface elements positioned on the exterior surface of at least one of the first plate or the second plate is configured to extend into the at least one opening on the first plate or the second plate that is positioned opposite of the one or more textured surface elements. The first plate and the second plate are porous, such that each of the first plate and the second plate include a plurality of openings that are in fluid communication with one another. At least one of the funnel or the enclosure is in fluid communication with a source of fluid of the medical device, and a fluid received at the funnel or the enclosure from the source of fluid is configured to move at least one of the first plate or the second plate relative to the other. The funnel is configured to guide the fluid toward the agent received therein to agitate the agent within the funnel. The funnel is in fluid communication with a delivery conduit of the medical device, and configured to guide a mixture of the fluid and the agent received from the funnel toward the delivery conduit. The valve assembly further includes an actuator coupled to at least one of the enclosure or the funnel, wherein the actuator is configured to move at least one of the first plate or the second plate relative to the other.

Also disclosed herein is an exemplary valve assembly for a medical device which includes an enclosure for storing an agent, the enclosure including a first end; a funnel positioned downstream of the enclosure for receiving the agent from the enclosure, the funnel including a second end positioned adjacent to the first end; and a porous mesh positioned between the first end and the second end, wherein at least a portion of the agent stored in the enclosure is received on the porous mesh; wherein the porous mesh is configured to move relative to the enclosure and the funnel to displace the agent stored in the enclosure for release into the funnel.

The disclosed exemplary valve assembly may include any of the following features. At least one of the funnel or the enclosure is in fluid communication with a source of fluid of the medical device, and a fluid received at the funnel or the enclosure from the source of fluid is configured to move the porous mesh. The valve assembly further including an actuator coupled to the porous mesh, wherein the actuator is configured to move the porous mesh relative to the enclosure and the funnel. The porous mesh is configured to rotate, translate laterally, or translate vertically relative to the enclosure and the funnel.

Further disclosed yet is another exemplary valve assembly for a medical device which includes an enclosure including: a chamber for storing an agent; a first funnel positioned downstream of the chamber for receiving the agent from the enclosure, the first funnel including a first porous surface; and a second funnel positioned downstream of the first funnel for receiving the agent from the first funnel, the second funnel including a second porous surface; an inlet that is in fluid communication with the enclosure and a source of fluid of the medical device; an outlet that is in fluid communication with the enclosure and a delivery conduit of the medical device; and wherein the first funnel is configured to receive fluid from the inlet via the first porous surface to agitate the agent received therein from the enclosure, and guide the agitated agent towards the second funnel, the second funnel is configured to receive fluid from the inlet via the second porous surface to agitate the agent received therein from the first funnel, and guide the agitated agent towards the outlet.

It may be understood that both the foregoing general description and the following detailed description are exemplary and explanatory only and are not restrictive of the invention, which is defined by the appended set of claims. As used herein, the terms "comprises," "comprising," "includes," "including," or any other variation thereof, are intended to cover a non-exclusive inclusion, such that a process, method, article, device, assembly, or apparatus that comprises a list of elements does not include only those elements, but may include other elements not expressly listed or inherent to such process, method, article, or apparatus. The term "diameter" may refer to a width where an element is not circular. The term "top" refers to a direction or side of a device relative to its orientation during use, and the term "bottom" refers to a direction or side of a device relative to its orientation during use that is opposite of the "top." The term "exemplary" is used in the sense of "example," rather than "ideal." The term "approximately," or like terms (e.g., "substantially"), includes values +/- 10% of a stated value.

### BRIEF DESCRIPTION OF THE DRAWINGS

The accompanying drawings, which are incorporated in and constitute a part of this specification, illustrate aspects of this disclosure and together with the description, serve to explain the principles of the disclosure.
FIG. 1 shows an exemplary delivery device.
FIG. 2 shows a schematic view of an exemplary valve assembly of the delivery device of FIG. 1.
FIG. 3 shows a partial side view of another exemplary valve assembly of the delivery device of FIG. 1.
FIG. 4 shows a partial side view of another exemplary valve assembly of the delivery device of FIG. 1.
FIG. 5 shows a partial side view of another exemplary valve assembly of the delivery device of FIG. 1.
FIG. 6 shows a partial side view of another exemplary valve assembly of the delivery device of FIG. 1.
FIG. 7A shows a partial side view of the valve assembly of FIG. 6 in a first position.
FIG. 7B shows a partial side view of the valve assembly of FIG. 6 in a second position.
FIG. 8 shows a partial side view of another exemplary valve assembly of the delivery device of FIG. 1.
FIG. 9 shows a partial side view of another exemplary valve assembly of the delivery device of FIG. 1.
FIG. 10A shows a partial side view of another exemplary valve assembly of the delivery device of FIG. 1 in a first position.
FIG. 10B shows a partial side view of the valve assembly of FIG. 10A in a second position.

### DETAILED DESCRIPTION

Embodiments of this disclosure relate to dispensing devices having valve assemblies for selectively releasing an agent (e.g., a powdered agent) to a site of a medical procedure. The valve assembly may include a series of funnels for fluidly coupling a pressurized medium source (e.g., a gas canister), from which the pressurized fluid (e.g., a gas) may be released, with an enclosure storing the agent. The agent may be received within the enclosure of the dispensing device, and in selective fluid communication with the pressurized fluid via the series of funnels. Accordingly, when the agent is received within the one or more funnels, the pressurized fluid that is selectively guided toward the funnels from the pressurized fluid source may travel toward and enter the funnels via a porous insert to agitate the agent prior to delivery to a target site of the medical procedure. Aspects of the dispensing device and valve assembly, such as the series of funnels, may facilitate a fluidization of the agent with the flow of pressurized fluid prior to the agent being delivered. The dispensing devices and valve assemblies may assist in selectively controlling the flow of pressurized fluid to help, for example, to help prevent or minimize clogging during delivery.

Other embodiments of the valve assembly may include respective surface plates at an interface between the enclosure and at least one funnel. Each of the surface plates may include one or more openings to facilitate a release of the agent from the enclosure and receipt of the agent in the funnel. At least one of the surface plates may include one or more textured surface elements configured to interact with the opposing plate to facilitate release of the agent from the enclosure and into the funnel. The textured surface elements of one surface plate may abut against the opposing surface plate to cause the agent stored in enclosure to move by displacing and/or dislodging the particles from an initial, rest state. Accordingly, when the agent is released from within the enclosure in response to a resulting movement caused by the engagement of the surface plates, and specifically the textured surface element, the agent enters the funnel to agitate the agent with pressurized fluid prior to delivery to a target site of the medical procedure. Aspects of the dispensing device and valve assembly, such as the surface plates and textured surface elements, may facilitate a fluidization of the agent with the flow of pressurized fluid prior to the agent being delivered, which may assist in selectively controlling the flow of pressurized fluid, for example, to help to prevent or minimize clogging during delivery.

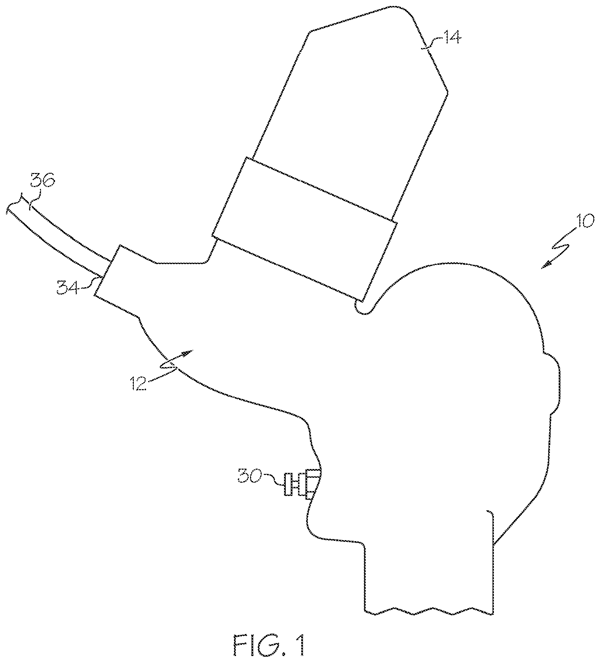

FIG. 1 shows a delivery device 10, which may be an agent (e.g., powder) delivery device. Delivery device 10 may include a handle body 12. Handle body 12 may include, or may be configured to receive, an enclosure 14 (or other source or container) storing a material (e.g., an agent). Enclosure 14 may be coupled to handle body 12 for providing the agent to handle body 12. In some aspects, a lid/enclosure of the agent may be screwed onto, or otherwise coupled to, enclosure 14 for supplying the agent to enclosure 14. The agent may be, for example, a powdered agent, such as a hemostatic agent. The agent may alternatively be another type of agent or material, or form of agent (e.g., a liquid or gel agent), and may have any desired function. Enclosure 14 may be removably attached to other components of delivery device 10, including components of handle body 12.

Handle body 12 may have a variety of features, to be discussed in further detail herein. U.S. Patent Application No. 16/589,633, filed October 1, 2019, published as U.S. Patent Application Publication No. 2020/0100986 A1 on April 2, 2022, discloses features of exemplary delivery devices and systems. The features of this disclosure may be combined with any of the features described in the above-referenced application. The features described herein may be used alone or in combination and are not mutually exclusive. Like reference numbers and/or terminology are used to denote similar structures, when possible.

Still referring to FIG. 1, delivery device 10 may include an actuation mechanism 30 used to activate flow of a pressurized fluid (e.g., gas) from a pressurized medium source in fluid communication with delivery device 10. Actuation mechanism 30 may be selectively actuated (e.g., manually depressible) or otherwise moved or actuated to control delivery of a material (e.g., a powdered agent) and pressurized fluid. Actuation mechanism 30 may include one or more actuation elements, such as, for example, a button, a slider, a lever, a trigger, a dial, and various other suitable actuators. The pressurized fluid alone, or a combination of a powdered agent and the pressurized fluid, may be delivered from an outlet 34 of handle body 12. Outlet 34 may be in fluid communication with a catheter 36 or another delivery conduit for delivering the combination of agent and fluid to a desired location within a body lumen of a patient.

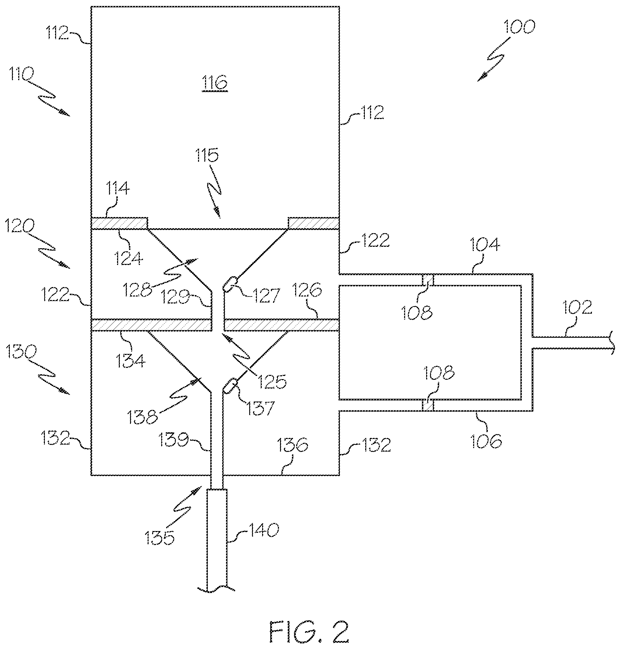

FIG. 2 shows aspects of an exemplary valve assembly 100. Valve assembly 100 may be at least partially housed within one or more of handle body 12 or enclosure 14 of delivery device 10 (see FIG. 1). Actuation mechanism 30 may be configured to selectively deliver a pressurized fluid from the pressurized fluid source (not shown) of delivery device 10 to valve assembly 100.

Valve assembly 100 may include a first enclosure 110, a second enclosure 120, and a third enclosure 130. First enclosure 110 may include at least a pair of sidewalls 112 and a lower wall 114 defining a chamber 116 for storing an agent. Second enclosure 120 may include at least a pair of sidewalls 122, an upper wall 124, and a lower wall 126 defining a cavity for housing a first funnel 128. Third enclosure 130 may include at least a pair of sidewalls 132, an upper wall 134, and a lower wall 136 defining a cavity for housing a second funnel 138.

In the example, at least a portion of first funnel 128 may be coupled to each of upper wall 124 and lower wall 126 along opposing ends of first funnel 128, such that first funnel 128 may be fluidly sealed within second enclosure 120. At least a portion of second funnel 138 may be coupled to each of upper wall 134 and lower wall 136 along opposing ends of second funnel 138, such that second funnel 138 may be fluidly sealed within third enclosure 130. As described herein, first funnel 128 and second funnel 138 may be in fluid communication with the pressurized fluid source via a porous insert positioned along a sidewall of the respective funnels.

Each of first enclosure 110, second enclosure 120, and third enclosure 130 may be coupled to at least another one of the enclosures of valve assembly 100. For example, first enclosure 110 may be positioned upstream from, and coupled to, second enclosure 120 along lower wall 114. Second enclosure 120 may be positioned downstream from, and coupled to, first enclosure 110 along upper wall 124. Second enclosure 120 may be further coupled to, and positioned upstream from, third enclosure 130 along lower wall 126. Third enclosure 130 may be positioned downstream from, and coupled to, second enclosure 120 along upper wall 134.

Still referring to FIG. 2, each of first enclosure 110, second enclosure 120, and third enclosure 130 may be in fluid communication with one another. In the example, lower wall 114 and upper wall 124 may collectively define a first opening 115 between first enclosure 110 and second enclosure 120, such that first enclosure 110 (and specifically chamber 116) may be in fluid communication with second enclosure 120 (and specifically first funnel 128) via first opening 115. Lower wall 126 and upper wall 134 may collectively define a second opening 125 between second enclosure 120 and third enclosure 130, such that second enclosure 120 (and specifically first funnel 128) may be in fluid communication with third enclosure 130 (and specifically second funnel 138) via second opening 125.

In the example, first opening 115 may have a greater diameter than second opening 125. As described in detail herein, a relative size of first opening 115 and second opening 125 may control a flow of the agent from first enclosure 110 to second enclosure 120, and a flow of the agent and pressurized fluid from second enclosure 120 and third enclosure 130, respectively. In other embodiments, first opening 115 may be relatively smaller and/or the same size as second opening 125. In further embodiments, one or more of first opening 115 and/or second opening 125 may be omitted entirely such that first enclosure 110, second enclosure 120, and third enclosure 130 may be in fluid communication with one another via various other suitable mechanisms, such as a conduit, a tube, a channel, etc.

Lower wall 136 of third enclosure 130 may include a third opening 135 for fluidly coupling third enclosure 130 (and specifically second funnel 138) to a tubing 140 of valve assembly 100. In some embodiments, at least a portion of second funnel 138 may extend through third opening 135 to fluidly couple with tubing 140, while in other embodiments tubing 140 may extend into third enclosure 130 via third opening 135 to fluidly couple with second funnel 138. Tubing 140 may be in fluid communication with outlet 34 of handle body 12 (see FIG. 1), such that tubing 140 may be fluidly coupled to catheter 36. As described herein, tubing 140 may be configured to deliver a mixture of agent and fluid received from first enclosure 110, second enclosure 120, and third enclosure 130 to a desired location within a body lumen of a patient via catheter 36. In some embodiments, tubing 140 may be flexible.

Still referring to FIG. 2, first funnel 128 may be disposed within second enclosure 120 between upper wall 124 and lower wall 126, and second funnel 138 may be disposed within third enclosure 130 between upper wall 134 and lower wall 136. First funnel 128 may include at least one insert 127 positioned along at least a portion (e.g., a sidewall) of first funnel 128, and second funnel 138 may include at least one insert 137 positioned along at least a portion (e.g., a sidewall) of second funnel 138. Each of the inserts 127, 137 may include a porous mesh that is configured to allow pressurized fluid received from the pressurized fluid source to pass therethrough and into the respective funnel 128, 138, while inhibiting an agent received within the funnel 128, 138 from exiting.

As described herein, each of second enclosure 120 and third enclosure 130 may be in fluid communication with a pressurized medium source of delivery device 10. A pressurized fluid received in each enclosure 120, 130 (from the pressurized medium source) may pass through the respective funnels 128, 138 via the corresponding insert 127, 137 to agitate the agent received therein. Although each of funnels 128, 138 are shown and described herein as including one insert, it should be appreciated that funnels 128, 138 may include additional inserts without departing from a scope of this disclosure.

Still referring to FIG. 2, second enclosure 120 may further include a first outlet channel 129 coupled to and extending outwardly from first funnel 128, and specifically downstream from first funnel 128. First outlet channel 129 may be fluidly coupled to first opening 115, such that a mixture of agent received in first funnel 128 (e.g., from first enclosure 110) and pressurized fluid received in first funnel 128 (e.g., from the pressurized medium source) may be mixed within first funnel 128 and guided through first outlet channel 129 for delivery into third enclosure 130 via second opening 125. In some embodiments, first outlet channel 129 may be coupled to a portion (e.g., a bottom wall) of first funnel 128, while in other embodiments first outlet channel 129 may be integral (i.e., integrally formed) with first funnel 128. In further embodiments, first outlet channel 129 may be omitted entirely such that first funnel 128 may be coupled directly to second opening 125.

Third enclosure 130 may include a second outlet channel 139 extending outwardly from second funnel 138, and specifically downstream from second funnel 138. Second outlet channel 139 may be fluidly coupled to third opening 135, such that a mixture of agent received in second funnel 138 (e.g., from second enclosure 120) and pressurized fluid received in second funnel 138 (e.g., from the pressurized medium source) may be mixed within second funnel 138 and guided through second outlet channel 139 for delivery to tubing 140 via third opening 135. In some embodiments, second outlet channel 139 may be coupled to a portion (e.g., a bottom wall) of second funnel 138, while in other embodiments second outlet channel 139 may be integral (i.e., integrally formed) with second funnel 138. In further embodiments, second outlet channel 139 may be omitted entirely such that second funnel 138 may be coupled directly to third opening 135 and/or tubing 140.

Still referring to FIG. 2, valve assembly 100 may include a fluidics channel 102 that is in fluid communication with the pressurized fluid source (not shown) of delivery device 10. Fluidics channel 102 may include a first tube 104 that is fluidly coupled to second enclosure 120, and a second tube 106 that is fluidly coupled to third enclosure 130. Each of the tubes 104, 106 may be portions of fluidics channel 102 that split from a main portion of fluidics channel 102 (i.e., via a bifurcation). As described herein, valve assembly 100 may be configured to simultaneously deliver the pressurized fluid to first tube 104 and second tube 106 via fluidics channel 102.

Each of first tube 104 and second tube 106 may include at least one restrictor mechanism 108 that is configured to control a flow rate of the pressurized fluid passing therethrough. For example, restrictor mechanisms 108 may be configured to reduce a pressure of the pressurized fluid received from the pressurized medium source of delivery device 10. In some embodiments, restrictor mechanisms 108 may be selectively adjustable to control the flow rate of pressurized fluid based on a user-defined pressure. Restrictor mechanism 108 may include various suitable devices, such as, for example, a metal plug having at least one opening, in which a size of the opening may determine the flow rate of fluid passing therethrough. In other embodiments, first tube 104 and/or second tube 106 may include two or more restrictor mechanisms 108 disposed therein, in which a quantity of restrictor mechanisms 108 may further determine the flow rate of the fluid passing therethrough.

In the example, first tube 104 may be fluidly coupled to second enclosure 120 along a portion of sidewall 122 that is positioned adjacent to insert 127 of first funnel 128. Stated differently, first tube 104 may be positioned relative to second enclosure 120 such that the pressurized fluid received within second enclosure 120 via first tube 104 may be guided in a direction toward a position of insert 127 on first funnel 128. In this instance, the pressurized fluid from first tube 104 may be directed toward insert 127 and received within first funnel 128 for agitating an agent received therein from first enclosure 110.

Still referring to FIG. 2, second tube 106 may be fluidly coupled to third enclosure 130 along a portion of sidewall 132 that is positioned adjacent to insert 137 of second funnel 138. Stated differently, second tube 106 may be positioned relative to third enclosure 130 such that the pressurized fluid received within third enclosure 130 via second tube 106 may be guided in a direction toward a position of insert 137 on second funnel 138. In this instance, the pressurized fluid from second tube 106 may be directed toward insert 137 and received within second funnel 138 for agitating an agent received therein from second enclosure 120.

In exemplary use, the agent stored in chamber 116 may exit first enclosure 110 at first opening 115 via gravitational forces, such that the agent may be received in second enclosure 120 at second funnel 128. As described above, a size of first opening 115 may at least partially determine a flow rate of the agent exiting chamber 116 into first funnel 128. In response to an actuation of actuation mechanism 30 (see FIG. 1), a pressurized fluid from the pressurized fluid source of delivery device 10 may be delivered through valve assembly 100 via fluidics channel 102.

At least a first portion of the pressurized fluid may be guided into second enclosure 120 via first tube 104, and at least a second portion of the pressurized fluid may be guided into third enclosure 130 via second tube 106. A flow rate of the pressurized fluid may be controlled by the one or more restrictor mechanisms 108 disposed within each of first tube 104 and second tube 106. As described above, with first funnel 128 coupled to upper wall 124 and first outlet channel 129 at opposing ends, and first outlet channel 129 further coupled to lower wall 126, first funnel 128 may be fluidly sealed within second enclosure 120. Accordingly, the first portion of pressurized fluid may only enter first funnel 128 via the at least one insert 127.

Still referring to FIG. 2, the first portion of pressurized fluid may at least partially agitate the agent received within first funnel 128 prior to guiding the agent toward third enclosure 130 via first outlet channel 129. With a mixture of the agent and pressurized fluid received in second enclosure 120, and specifically first funnel 128, valve assembly 100 may be configured to at least partially mix the agent and pressurized fluid within first funnel 128 prior to delivery to second funnel 138. In this instance, second enclosure 120 may be configured to control a flow rate and/or volume of the agent guided into third enclosure 130. For example, a size (i.e., a cross-sectional width) of first funnel 128 and/or first outlet channel 129 may control a flow rate and/or volume of agent guided into third enclosure 130. By limiting the flow and/or volume of agent delivered from second enclosure 120 to third enclosure 130, valve assembly 100 may be configured to help prevent second funnel 138 from clogging.

With second funnel 138 coupled to upper wall 134 and second outlet channel 139 at opposing ends, and second outlet channel 139 further coupled to lower wall 136, second funnel 138 may be fluidly sealed within third enclosure 130. Accordingly, the second portion of pressurized fluid may only enter second funnel 138 via the at least one insert 137. The second portion of pressurized fluid may fluidize (or otherwise further agitate) the agent received from second enclosure 120 within second funnel 138 prior to guiding the fluidized agent toward tubing 140, for example, via second outlet channel 139.

In this instance, third enclosure 130 may be configured to fluidize the at least partially agitated agent and control a flow rate and volume of the agent guided into tubing 140. In this instance, valve assembly 100 may be configured to provide multiple stages of fluidization, such as at second enclosure 120 and at third enclosure 130. In the embodiment, an incoming flow rate of the agent received in second funnel 138 may be substantially similar to an outgoing flow rate of the agent exiting second funnel 138. For example, a size (i.e., a cross-sectional width) of second funnel 138 and/or second outlet channel 139 may control a flow rate and/or volume of agent guided into tubing 140. By limiting the flow and/or volume of agent delivered from third enclosure 130 to tubing 140, valve assembly 100 may be configured to help prevent tubing 140 from clogging.

In other embodiments, valve assembly 100 may be configured to provide a single stage of fluidization. In this instance, valve assembly 100 may be configured such that the first portion of pressurized fluid received within second enclosure 120 (via first tube 104) may have a flow rate sufficient for moving the agent received in first funnel 128 toward second funnel 138 without substantially agitating the agent. Stated differently, first funnel 128 may be configured to guide the agent received therein from first enclosure 110 to third enclosure 130 without substantially agitating and/or fluidizing the agent. For example, valve assembly 100 may control the flow rate of the first portion of pressurized fluid received in second enclosure 120 via the corresponding restrictor mechanism(s) 108 disposed within first tube 104 to help prevent the agent in first funnel 128 from being agitated.

Accordingly, the agent received in third enclosure 130, and specifically in second funnel 138, may be initially agitated therein by the second portion of pressurized fluid from second tube 106. In this instance, the second portion of pressurized fluid may have a greater flow rate relative to the first portion of pressurized fluid, with the flow rate of the second portion of pressurized fluid sufficiently agitating and/or fluidizing the agent in second funnel 138 prior to delivery to tubing 140.

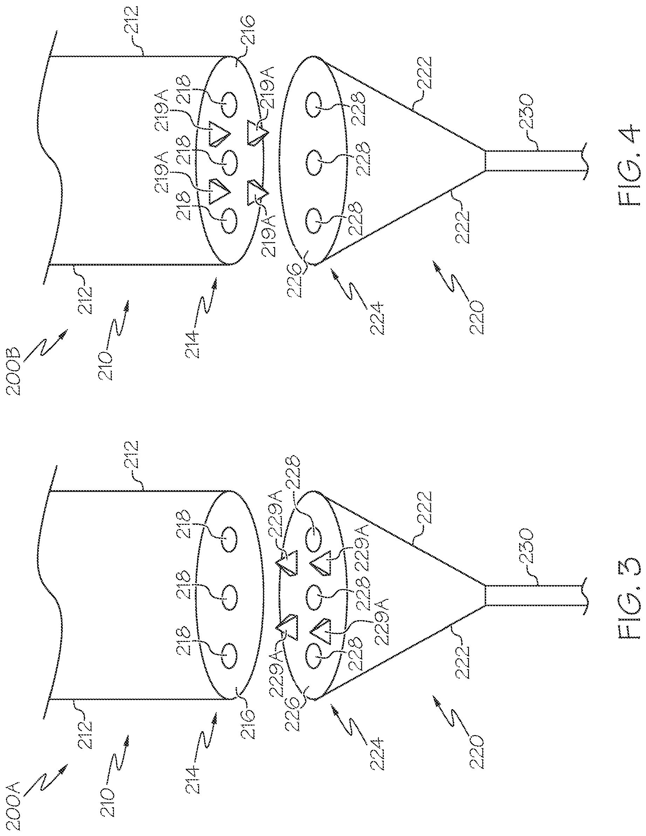

Referring now to FIGS. 3-7B, aspects of other exemplary valve assemblies 200A-200E are shown. Valve assemblies 200A-200E may be housed within handle body 12 and/or enclosure 14 (see FIG. 1). In some embodiments, valve assemblies 200A-200E may be selectively actuated (e.g., moved) by actuation mechanism 30. In this instance, actuation mechanism 30 may be configured to deliver pressurized fluid from the pressurized fluid source to valve assembly 200A-200E (e.g., via fluidics channel 102 in FIG. 2), and provide for a corresponding movement of one or more components of valve assembly 200A-200E. Accordingly, actuation mechanism 30 may be configured to simultaneously establish fluid communication between the pressurized fluid source and valve assembly 200A-200E and also control movement of valve assembly 200A-200E. As described herein, actuation of actuation mechanism 30 may provide a corresponding movement of an enclosure and/or funnel to control release of an agent.

Referring initially to FIG. 3, valve assembly 200A may include an enclosure 210 that is substantially similar to at least first enclosure 110 shown and described above. Enclosure 210 may include at least a pair of sidewalls 212 and a lower (open) end 214 that collectively define a chamber for storing an agent. Enclosure 210 may be generally cylindrical, and the pair of sidewalls 212 may be generally circular. Valve assembly 200A may further include a first plate 216 coupled to lower end 214, with first plate 216 configured to at least partially enclose enclosure 210 at lower end 214. In some embodiments, first plate 216 may be separate from enclosure 210, while in other embodiments first plate 216 may be integral (i.e., integrally formed) with enclosure 210. In the example, first plate 216 may include one or more openings 218 that facilitate access to the chamber of enclosure 210, such as for releasing the agent stored therein.

Valve assembly 200A may include a funnel 220 that is substantially similar to funnels 128, 138 shown and described above with respect to valve assembly 100. Funnel 220 may include at least a sidewall 222, an upper (open) end 224, and an outlet channel 230 extending outwardly (e.g., downward) from sidewall 222. Outlet channel 230 may be coupled to sidewall 222, integral with sidewall 222, and/or omitted entirely. Valve assembly 200A may further include a second plate 226 coupled to upper end 224, with second plate 226 configured to at least partially enclose an internal cavity of funnel 220 at upper end 224. In the example, second plate 226 may include one or more openings 228 that facilitate access to the inner cavity of funnel 220, such as for receiving the agent from enclosure 210. In some embodiments, second plate 226 may be separate from funnel 220, while in other embodiments second plate 226 may be integral (i.e., integrally formed) with funnel 220.

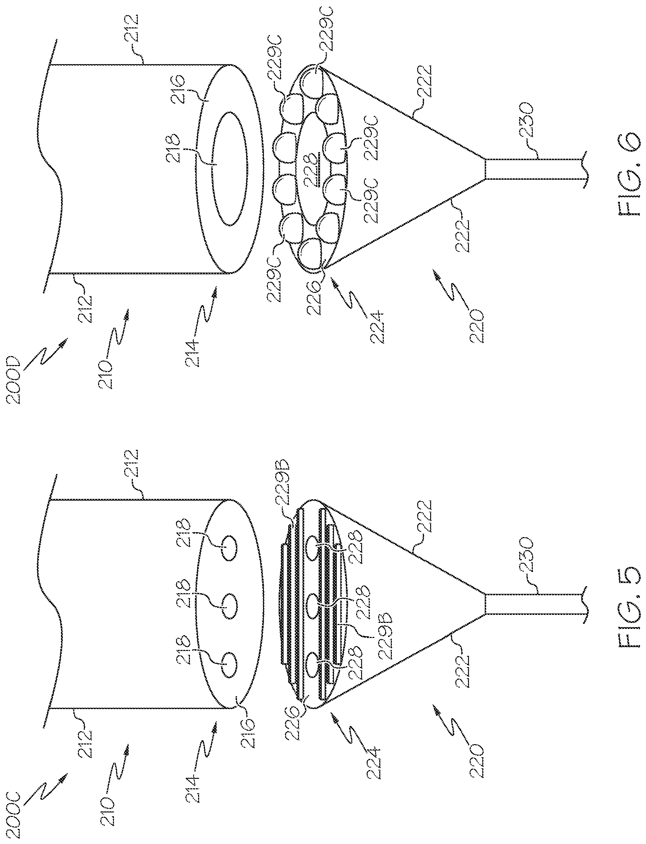

In the example, first plate 216 may include three openings 218, and second plate 226 may include three openings 228. In other examples, first plate 216 and/or second plate 226 may include additional and/or fewer openings (see FIG. 6). Although first plate 216 and second plate 226 are shown and described herein as having a corresponding number (e.g., a same number) of openings, it should be appreciated that each plate 216, 226 may have a different number of openings relative to the other without departing from a scope of this disclosure. First plate 216 may be configured to form a face of enclosure 210 at lower end 214, and second plate 226 may be configured to form a corresponding face of funnel 220 at upper end 224. Each of the faces formed by first plate 216 and second plate 226 may define a respective interface of enclosure 210 and funnel 220, respectively.

In some embodiments, one or more of enclosure 210 and/or funnel 220 may include at least one textured surface member, element, or feature positioned along the respective face, with the textured surface member being configured to facilitate an agitation and/or release of the agent stored in enclosure 210 for receipt in funnel 220. In other words, the at least one textured surface member may provide a physical obstruction, barrier, and/or impediment between enclosure 210 and funnel 220 that is configured to generate a turbulence and/or disruption at the interface connection between enclosure 210 and funnel 220 upon movement of the textured surface member. The resulting turbulence and/or disruption may help to displace and/or dislodge the agent stored in enclosure 210, to facilitate movement of the agent into funnel 220. Stated differently, the at least one textured surface member may agitate the agent within enclosure 210, thereby urging the agent out of enclosure 210 and into funnel 220. In some embodiments, the at least one textured surface member may be positioned along at least one of first plate 216 or second plate 226.

For example, as seen in FIG. 3, valve assembly 200A may include a plurality of textured surface members 229A positioned on second plate 226. The plurality of textured surface members 229A may be sized, shaped, and/or otherwise configured as sharp protrusions (e.g., pyramidal, conical, etc. protrusions) extending outwardly from an exterior surface of second plate 226. Although four textured surface members 229A are shown at particular positions along second plate 226, it should be appreciated that second plate 226 may include additional and/or fewer textured surface members 229A at various other relative locations along second plate 226. It should further be understood that each of the plurality of textured surface members 229A positioned on second plate 226 may have different shapes, sizes, and/or configurations relative to one another.

The plurality of textured surface members 229A may be configured to interface with first plate 216 when enclosure 210 is coupled to funnel 220. For example, textured surface members 229A may abut against, engage, and/or otherwise contact the exterior surface of first plate 216 when lower end 214 is coupled to upper end 224. In some embodiments, at least one of first plate 216 or second plate 226 may be configured to move relative to the other. For example, first plate 216 and/or second plate 226 may be configured to translate laterally, translate longitudinally, pivot, and/or rotate relative to one another. In other embodiments, at least one of enclosure 210 or funnel 220 may be configured to move relative to the other, thereby moving the corresponding first plate 216 or second plate 226 coupled thereto, respectively.

In response to a movement of first plate 216 and/or second plate 226, the plurality of textured surface members 229A may be configured to abut against first plate 216 and generate a vibrating motion within enclosure 210. The resulting vibration may help to displace and/or dislodge the agent stored within enclosure 210. In some embodiments, movement of first plate 216 and/or second plate 226 may be caused by a pressurized fluid received from the pressurized fluid source of delivery device 10 (FIG. 1). For example, at least one of enclosure 210 or funnel 220 may be in fluid communication with the pressurized fluid source (e.g., as discussed with respect to fluidics channel 102 in FIG. 2), and the pressurized fluid received therein may move at least one of first plate 216 or second plate 226 relative to the other.

In other embodiments, valve assembly 200A may include an actuator (not shown) coupled to at least one of enclosure 210 or funnel 220. The actuator may be configured to move at least one of first plate 216 or second plate 226 relative to the other. For example, the actuator may include a cable, a wire, a rod, a shaft, or various other suitable devices for applying a physical force (e.g., a pulling force, a pushing force, etc.) onto one or more of first plate 216 or second plate 226. Actuation of the actuator may move textured surface members 229A and cause a vibration of the agent stored within enclosure 210. The actuator may be communicatively coupled to actuation mechanism 30, such that actuation of actuation mechanism 30 may provide for a movement of the actuator and a corresponding movement of textured surface members 229A.

Still referring to FIG. 3, upon moving one or more of first plate 216 and/or second plate 226, the plurality of textured surface members 229A may engage against an exterior surface of first plate 216 and/or the plurality of openings 218 positioned on first plate 216. In the example, the plurality of textured surface members 229A may be configured to continuously move relative to first plate 216 while actuation mechanism 30 remains actuated, thereby providing an uninterrupted agitation of the agent within enclosure 210. Upon agitating the agent within enclosure 210, the agitated agent may be released from enclosure 210 via openings 218 and received within funnel 220 via openings 228.

In other embodiments, the valve assembly may include a plurality of textured surface members positioned on first plate 216 in lieu of and/or in addition to second plate 226. For example, as seen in FIG. 4, an exemplary valve assembly 200B may include a plurality of textured surface members 219A positioned along an exterior surface of first plate 216. Valve assembly 200B may be configured similar to valve assembly 200A except for the differences explicitly described herein, such that like reference numerals are used to identify like components. In this instance, textured surface members 219A may be configured to engage an exterior surface of second plate 226 and/or the plurality of openings 228 in response to a movement of textured surface members 219A.

In the example, the plurality of textured surface members 219A may be sized, shaped, and/or otherwise configured similar to textured surface members 229A shown and described above, e.g., as sharp protrusions extending outwardly from the exterior surface of first plate 216. It should be understood that valve assembly 200B may be configured and operable to agitate the agent within enclosure 210 in a substantially similar manner as described above irrespective of whether first plate 216, second plate 226, or both include textured surface members. Although the textured surface members have been shown and described herein as sharp protrusions, it should be appreciated that valve assembly 200B may include textured surface members of various suitable sizes, shapes, and/or configurations. For example, the textured surface members may include a ridge, a rounded protrusion, a projection, a bump, a rib, a tooth, a baffle, a dimple, a recess, a cavity, or other features capable of forming a physical structure.

For example, as seen in FIG. 5, an exemplary valve assembly 200C may include a plurality of textured surface members 229B, for example, in the form of elongated ridges extending across the exterior surface of second plate 226. Valve assembly 200C may be configured similar to valve assembly 200A, 200B except for the differences explicitly described herein, such that like reference numerals are used to identify like components. The elongated ridges may have similar or varying cross-sectional profiles, such as similar or varying longitudinal lengths, lateral widths, and/or heights relative to one another. Additionally and/or alternatively, valve assembly 200C may include a plurality of textured surface members in the form of elongated ridges on first plate 216 for interacting with second plate 226. In the example, the plurality of textured surface members 229B may be positioned parallel to one another, and spaced apart from openings 228. The plurality of textured surface members 229B may be positioned on opposing sides of openings 228 relative to the exterior surface of second plate 226.

In another example, as seen in FIG. 6, an exemplary valve assembly 2000 may include a plurality of textured surface members 229C, for example, in the form of bulbous extensions, bumps, tips, etc. extending across the exterior surface of second plate 226. Valve assembly 200D may be configured similar to valve assembly 200A, 2008, 200C except for the differences explicitly described herein, such that like reference numerals are used to identify like components. Second plate 226 may include a single opening 228, for example, positioned along a central portion of second plate 226. In this instance, first plate 216 may include a corresponding single opening 218, for example, positioned along a central portion of first plate 216. The plurality of textured surface members 229C may be positioned in an annular array about a circumference of opening 218.

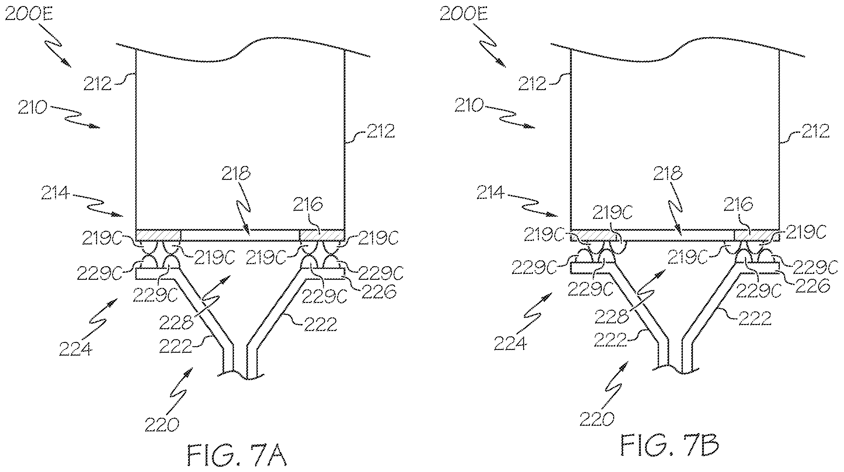

By way of further example, as shown in the cross-sectional side views of FIGS. 7A and 7B, an exemplary valve assembly 200E may include a plurality of textured surface members 219C on first plate 216 that correspond with the plurality of textured surface members 229C on second plate 226. Valve assembly 200E may be configured similar to valve assembly 200A, 200B, 200C, 200D except for the differences explicitly described herein, such that like reference numerals are used to identify like components. In this instance, textured surface members 219C may engage textured surface members 229C when at least one of first plate 216 or second plate 226 moves (e.g., translates, pivots, rotates, etc.) relative to the other.

In the example, each of the textured surface members 219C, 229C may move from a first position, in which the textured surface members 219C, 229C are positioned in linear abutment with one another (FIG. 7A), to a second position in which the textured surface members 219C, 229C may be at least partially offset and positioned in a lateral abutment with one another (FIG. 7B). By moving the textured surface members 219C, 229C repeatedly between the first and second positions, the agent stored in enclosure 210 may be agitated within enclosure 210 and released from opening 218 for receipt within opening 228. It should be appreciated that, but for the differences described above with respect to the various textured surface members, an exemplary use of valve assembly 200A-200E may remain substantially the same in FIGS. 3-7B.

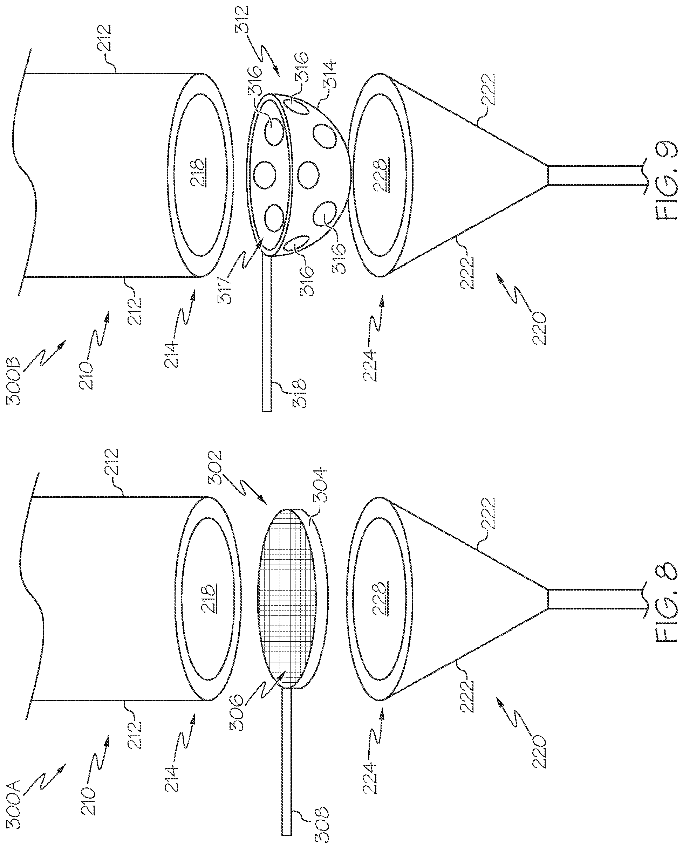

Referring now to FIGS. 8-9, aspects of additional exemplary valve assemblies 300A, 300B are shown. Valve assemblies 300A, 300B may be configured similar to valve assembly 200A-200E except for the differences explicitly described herein, such that like reference numerals are used to identify like components. Valve assembly 300A, 300B may be housed within handle body 12 and/or enclosure 14 (see FIG. 1). Valve assembly 300A, 300B may include a mesh assembly positioned between and coupled to enclosure 210 and funnel 220. As described herein, the mesh assembly may be configured to fluidly couple enclosure 210 to funnel 220, and further configured to move relative to at least one of enclosure 210 or funnel 220.

Referring specifically to FIG. 8, valve assembly 300A may include a mesh assembly 302 disposed between lower end 214 of enclosure 210 and upper end 224 of funnel 220. Mesh assembly 302 may include a planar body 304 and a porous mesh 306 coupled to planar body 304. Planar body 304 may be sized, shaped, and/or otherwise configured to fit between lower end 214 and upper end 224, and particularly within the corresponding openings 218, 228. In some embodiments, planar body 304 may have a disc-shaped, concave, and/or convex profile. Porous mesh 306 may be stretched over planar body 304, and configured to control a flow rate of the agent exiting enclosure 210 (via opening 218) and entering into funnel 220 (via opening 228). Stated differently, a porosity of porous mesh 306 (i.e., a size of the plurality of pores) may determine a flow rate of the agent being received within funnel 220 from enclosure 210.

Mesh assembly 302 may be configured to move relative to at least one of enclosure 210 or funnel 220. For example, mesh assembly 302 may be configured to translate laterally, translate longitudinally, rotate, and/or pivot relative to at least one of lower end 214 of enclosure 210 or upper end 224 of funnel 220. In some embodiments, mesh assembly 302 may be independently movable relative to each of enclosure 210 and funnel 220. In other embodiments, mesh assembly 302 may be fixed to at least one of enclosure 210 and/or funnel 220 such that mesh assembly 302 may be configured to move in response to a corresponding movement of enclosure 210 and/or funnel 220.

Still referring to FIG. 8, in response to a movement of mesh assembly 302, planar body 304 may be configured to abut against lower end 214 and generate a vibrating motion within enclosure 210, thereby helping to displace and/or dislodge the agent stored therein. In some embodiments, movement of mesh assembly 302 may be caused by a pressurized fluid received from the pressurized fluid source of delivery device 10 (FIG. 1), such as via fluidics channel 102. For example, at least one of enclosure 210 or funnel 220 may be in fluid communication with the pressurized fluid source, and the pressurized fluid received at enclosure 210 or funnel 220 may move mesh assembly 302, thereby causing planar body 304 to oscillate and/or vibrate against lower end 214.

In other embodiments, valve assembly 300A may include an actuator 308 coupled to planar body 304. Actuator 308 may be configured to move planar body 304 relative to one or more of enclosure 210 or funnel 220. For example, actuator 308 may include a cable, a wire, a rod, a shaft, or various other devices for applying a physical force (e.g., a pulling force, a pushing force, etc.) onto planar body 304, thereby generating movement of mesh assembly 302 against enclosure 210 and causing a vibration of the agent stored therein. Actuator 308 may be communicatively coupled to actuation mechanism 30 (see FIG. 1), such that actuation of actuation mechanism 30 may provide a movement of actuator 308 and a corresponding movement of mesh assembly 302. In other embodiments, actuator 308 may be communicatively coupled to a separate mechanism of delivery device 10 for actuating mesh assembly 302 independent of actuation mechanism 30.

Alternatively, referring now to FIG. 9, valve assembly 300B may include a mesh assembly 312 disposed between lower end 214 of enclosure 210 and upper end 224 of funnel 220. Mesh assembly 312 may be substantially similar to mesh assembly 302 except for the differences explicitly described herein, such that mesh assembly 312 may be configured and operable like mesh assembly 302. Mesh assembly 312 may include a rounded (e.g., circular) body 314 having a center opening 317 along a top portion of rounded body 314. Rounded body 314 may be porous and include a plurality of pores 316 that are configured to control a flow rate of the agent exiting enclosure 210 (via opening 218) and entering into funnel 220 (via opening 228). Stated differently, a porosity of rounded body 314 (i.e., a size, shape, quantity, and/or position of the plurality of pores 316) may determine a flow rate of the agent being received within funnel 220 from enclosure 210.

Center opening 317 may be sized, shaped, and/or otherwise configured to receive the agent within rounded body 314 from enclosure 210 (via opening 218), and rounded body 314 may be configured to at least partially maintain the agent therein prior to releasing the agent into funnel 220 (through opening 228) via the plurality of pores 316. In the example, rounded body 314 may have a generally circular shape. Rounded body 314 may be sized, shaped, and/or otherwise configured to fit between lower end 214 and upper end 224, and particularly within the corresponding openings 218, 228. In other examples, rounded body 314 may have various other three-dimensional shapes and/or cross-sectional profiles, such as rectangular, squared, cylindrical, oval, and more.

Still referring to FIG. 9, mesh assembly 312 may be configured to move relative to at least one of enclosure 210 or funnel 220. For example, mesh assembly 312 may be configured to translate laterally, translate longitudinally, rotate, and/or pivot relative to lower end 214 and/or upper end 224. Mesh assembly 312 may either be independently movable relative to enclosure 210 and funnel 220, or fixed to at least one such that mesh assembly 312 may be simultaneously movable with one of enclosure 210 and/or funnel 220. In response to a movement of mesh assembly 312, rounded body 314 may be configured to abut against lower end 214 and generate a vibrating motion within enclosure 210, thereby displacing and/or dislodging the agent.

Movement of mesh assembly 312 may be caused by pressurized fluid from the pressurized fluid source, or from an actuator 318 of mesh assembly 312. Actuator 318 may be coupled to rounded body 314, and configured to move rounded body 314 relative to enclosure 210 and/or funnel 220. Actuator 318 may be substantially similar to actuator 308 shown and described in detail above.

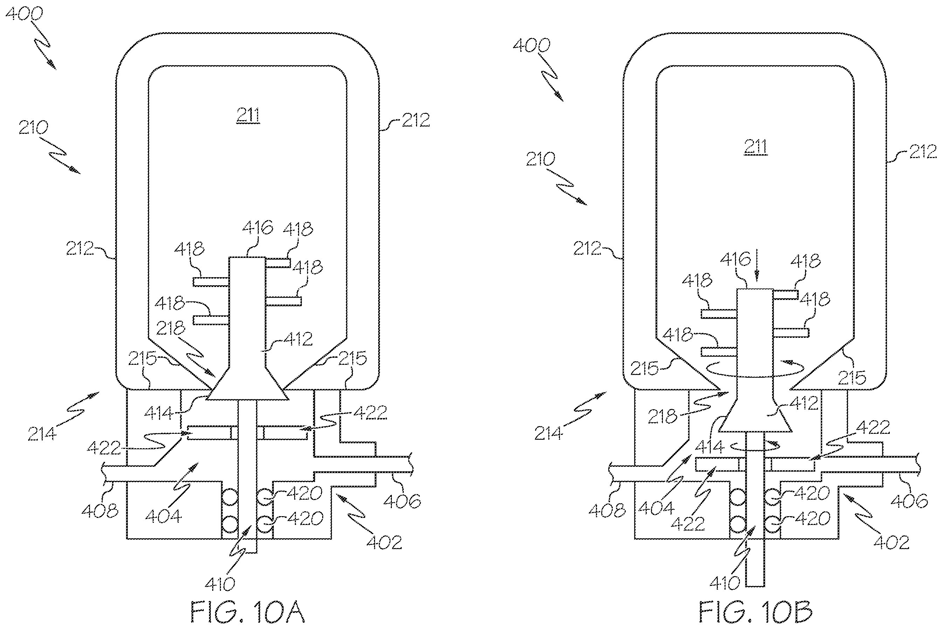

Referring now to the cross-sectional side views of FIGS. 10A-10B, aspects of another exemplary valve assembly 400 is shown. Valve assembly 400 may be configured similar to valve assembly 200A-200E except for the differences explicitly described herein, such that like reference numerals are used to identify like components. Valve assembly 400 may be housed within handle body 12 and/or enclosure 14 (see FIG. 1). In some embodiments, valve assembly 400 may be selectively actuated (e.g., moved) by actuation mechanism 30. In this instance, actuation mechanism 30 may be configured to deliver pressurized fluid from the pressurized fluid source to valve assembly 400, and provide for a corresponding movement of one or more components or portions of valve assembly 400. Accordingly, actuation mechanism 30 may be configured to simultaneously establish fluid communication between the pressurized fluid source and valve assembly 400 and control movement of valve assembly 400.

Valve assembly 400 may include a fixed body 402 and a movable body 412. Fixed body 402 may define a housing that is coupled to enclosure 210, such as along lower end 214. Fixed body 402 may include a channel 404 disposed between an inlet 406 and an outlet 408. Channel 404 may be in fluid communication with the pressurized fluid source via inlet 406, and with catheter 36 (FIG. 1) via outlet 408. Fixed body 402 may be in further fluid communication with enclosure 210, such that channel 404 may be fluidly coupled to a chamber 211 of enclosure 210, such as through opening 218 at lower end 214 of enclosure 210. As described herein, valve assembly 400 may be configured to selectively fluidly couple channel 404 to chamber 211 in response to moving movable body 412 between one or more positions.

Referring to FIG. 10A, movable body 412 may be at least partially received within, and configured to move (e.g., translate, rotate, etc.) relative to, fixed body 402 and enclosure 210. Valve assembly 400 may include a shaft 410 coupled to movable body 412, and extending through at least a portion of fixed body 402, including channel 404. With shaft 410 received through channel 404, valve assembly 400 may include one or more gaskets 420 (e.g., O-rings) disposed about shaft 410, for example, to help inhibit any fluid and/or agent received in channel 404 from exiting fixed body 402, for example, around shaft 410.

Movable body 412 may be movable (e.g., rotatable, translatable, etc.) relative to fixed body 402 and enclosure 210. Movable body 412 may be coupled to shaft 410, such that movement of movable body 412 may provide a corresponding movement of shaft 410, and vice versa. As described in detail below, movable body 412 may be configured to rotate, for example, in response to channel 404 receiving pressurized fluid from the pressurized fluid source. Furthermore, movable body 412 may be configured to translate in response to shaft 410 translating relative to fixed body 402. In some embodiments, shaft 410 may be configured to translate through fixed body 402 and relative to enclosure 210 in response to actuation of actuation mechanism 30. In other embodiments, shaft 410 may translate in response to the actuation of another actuator of delivery device 10.

Still referring to FIG. 10A, movable body 412 may have a longitudinal length defined between a first end 414 and a second end 416 that is opposite of first end 414. First end 414 may be positioned within channel 404, and second end 416 may be positioned within chamber 211. Valve assembly 300A may further include a plurality of paddles 422 coupled to, and extending laterally outward from, shaft 410. The plurality of paddles 422 may be positioned along shaft 410 relatively below movable body 412, and particularly first end 414. With shaft 410 disposed within channel 404, the plurality of paddles 422 may be configured to interact with the pressurized fluid received in channel 404 via inlet 406.

As described in detail herein, the plurality of paddles 422 may be sized, shaped, and/or otherwise configured to move (e.g., rotate) shaft 410 relative to fixed body 402, and thereby move movable body 412 relative to enclosure 210, in response to the pressurized fluid encountering paddles 422 within channel 404. Stated differently, the pressurized fluid traveling through channel 404 from inlet 406 to outlet 408 may contact and urge the plurality of paddles 422 into a rotational movement within channel 404, thereby causing shaft 410 and movable body 412 to simultaneously rotate (see FIG. 10B).

Still referring to FIG. 10A, valve assembly 400 may include a plurality of arms 418 coupled to, and extending laterally outward from, movable body 412, such as adjacent to second end 416. With second end 416 disposed within chamber 211, the plurality of arms 418 may be configured to contact the agent stored within enclosure 210. As described in detail herein, the plurality of arms 418 may be sized, shaped, and/or otherwise configured to agitate the agent within chamber 211 in response to a movement of movable body 412 relative to enclosure 210. In some embodiments, the plurality of arms 418 may be positioned in an alternating arrangement along a longitudinal length of movable body 412. The plurality of arms 418 may include rods having similar and/or varying sizes and/or shapes (e.g., longitudinal lengths, widths) relative to one another.

First end 414 may be sized, shaped, and/or otherwise configured to interface with lower end 214 of enclosure 210 when movable body 412 is in a first position (see FIG. 10A). Lower end 214 may be defined by one or more surfaces 215 of enclosure 210. In the example, the one or more surfaces 215 may be angled, and may at least partially define opening 218 of enclosure 210 at lower end 214. First end 414 may include one or more walls and/or surfaces that are configured to mate with the one or more surfaces 215, such that first end 414 may be configured to couple with lower end 214 when movable body 412 is in the first position relative to enclosure 210. In this instance, first end 414 engages with surfaces 215, and movable body 412 seals chamber 211 from channel 404, thereby fluidly decoupling or isolating fixed body 402 from enclosure 210 when movable body 412 is in the first position, as shown in FIG. 10A.

In an exemplary use, movable body 412 may be in the first position shown in FIG. 10A with first end 414 positioned against surfaces 215 at lower end 214, thereby closing opening 218. With opening 218 closed by movable body 412, chamber 211 may be fluidly decoupled or isolated from channel 404 such that the agent stored in chamber 211 may be inhibited from exiting enclosure 210 into channel 404. The plurality of paddles 422 may be positioned within channel 404 at a position relatively above inlet 406. In other words, the plurality of paddles 422 may be offset and/or misaligned with inlet 406, such that the plurality of paddles 422 may not be in a fluid flow path between inlet 406 and outlet 408.

Referring now to FIG. 10B, upon actuating actuation mechanism 30 or another actuator (not shown), shaft 410 may translate downward relative to fixed body 402 to move movable body 412 to a second position. In this instance, first end 414 of movable body 412 may disengage from surfaces 215 at lower end 214, thereby opening the opening 218. With opening 218 opened by movable body 412, chamber 211 may be fluidly coupled to channel 404 such that the agent stored in chamber 211 may exit enclosure 210 and be received into channel 404. The plurality of paddles 422 may be positioned within channel 404 at a position aligned with inlet 406 (i.e., between inlet 406 and outlet 408) such that paddles 422 may be positioned in the fluid flow path between inlet 406 and outlet 408.

Actuating actuation mechanism 30 may further deliver a pressurized fluid from the pressurized fluid source into channel 404 via inlet 406. With the plurality of paddles 422 positioned within the fluid flow path between inlet 406 and outlet 408, valve assembly 400 may be configured to rotate shaft 410 and movable body 412 in response to the pressurized fluid entering channel 404 and pushing paddles 422, In the example, the plurality of paddles 422 may be include various suitable sizes and/or shapes. For example, paddles 422 may be shaped in accordance with a propeller for maximizing capture of the pressurized fluid for rotating shaft 410 within channel 404. In another example, paddles 422 may be shaped in accordance with a turbine blade for inducing a downward motion to facilitate a downward release of the agent from enclosure 210 into channel 404.

Still referring to FIG. 10B, with movable body 412 coupled to shaft 410 along first end 414, movable body 412 may be configured to rotate with shaft 410. In addition or in alternative to a rotational movement, shaft 410 and movable body 412 may be configured to oscillate vertically. The plurality of arms 418 within enclosure 210 may be configured to help displace and/or dislodge the agent stored in chamber 211, thereby moving the agent into channel 404 via opening 218 (i.e., around first end 414). With the agent received within channel 404, the pressurized fluid may agitate the agent within fixed body 402 prior to guiding the agitated agent toward outlet 408 for delivery to a patient via catheter 36 (FIG. 1). In other embodiments, valve assembly 400 may be motorized such that shaft 410 and movable body 412 may be configured to translate and/or rotate in response to an activation of a motor, such as a battery operated motor. Alternatively, valve assembly 400 may include one or more coil springs that are pre-wound and configured to provide movement of shaft 410 and movable body 412. In this instance, the coil spring(s) may be selectively released to unwind, thereby causing movement of shaft 410 and movable body 412.

Upon terminating actuation of actuation mechanism 30 or the other actuator, delivery of the pressurized fluid into channel 404 may be terminated, thereby ceasing further rotation of paddles 422, shaft 410, and movable body 412. Further, shaft 410 may translate upward toward enclosure 210, thereby returning movable body 412 to the first position (FIG. 10A) with first end 414 engaged against surfaces 215 at lower end 214. In this instance, movable body 412 may fluidly decouple and/or isolate chamber 211 from channel 404, thereby inhibiting the agent stored in enclosure 210 from entering fixed body 402.

While principles of this disclosure are described herein with reference to illustrative examples for particular applications, it should be understood that the disclosure is not limited thereto. Those having ordinary skill in the art and access to the teachings provided herein will recognize additional modifications, applications, and substitution of equivalents all fall within the scope of the examples described herein. Accordingly, the invention is not to be considered as limited by the foregoing description, but only by the following claims.

## Claims

1. A valve assembly (200A-200E) for a medical device (10), comprising:
an enclosure (210) for storing an agent, the enclosure including a first end (214);
a funnel (220) positioned downstream of the enclosure for receiving the agent from the enclosure, the funnel including a second end (224) positioned adjacent to the first end;
a first plate (216) coupled to the enclosure at the first end, the first plate including at least one opening (218); and
a second plate (226) coupled to the funnel at the second end, the second plate including at least one opening (228) that is in fluid communication with the at least one opening of the first plate;
wherein the first plate and the second plate are positioned against one another, and an exterior surface of at least one of the first plate or the second plate includes one or more textured surface elements (219A, 219C, 229A, 229B, 229C) configured to displace the agent stored in the enclosure in response to at least one of the first plate or the second plate moving relative to one another;
wherein the one or more textured surface elements is configured to vibrate the enclosure, in response to at least one of the first plate or the second plate moving relative to one another, to displace the agent stored in the enclosure.

2. The valve assembly of claim 1, wherein the one or more textured surface elements includes a ridge, a protrusion, a projection, a bump, a rib, a tooth, a baffle, a dimple, a recess, or a cavity.

3. The valve assembly of any one of the preceding claims, wherein the first plate is configured to rotate or translate relative to the second plate.

4. The valve assembly of any one of the preceding claims, wherein the one or more textured surface elements is configured to agitate the agent in the enclosure and release the agitated agent from the enclosure and into the funnel in response to at least one of the first plate or the second plate moving relative to one another.

5. The valve assembly of any one of the preceding claims, wherein the exterior surface of each of the first plate and the second plate include one or more textured surface elements.

6. The valve assembly of claim 5, wherein the one or more textured surface elements positioned on the exterior surface of the first plate are configured to engage the one or more textured surface elements positioned on the exterior surface of the second plate, thereby displacing the agent stored in the enclosure.

7. The valve assembly of claim 6, wherein at least one of the first plate or the second plate is configured to translate in a longitudinal direction relative to one another when the one or more textured surface elements of the first plate engage the one or more textured surface elements of the second plate.

8. The valve assembly of any one of the preceding claims, wherein each of the first plate and the second plate are configured to move relative to one another to displace the agent stored in the enclosure for release into the funnel.

9. The valve assembly of any one of the preceding claims, wherein the one or more textured surface elements positioned on the exterior surface of at least one of the first plate or the second plate is configured to extend into the at least one opening on the first plate or the second plate that is positioned opposite of the one or more textured surface elements.

10. The valve assembly of any one of the preceding claims, wherein the first plate and the second plate are porous, such that each of the first plate and the second plate include a plurality of openings that are in fluid communication with one another.

11. The valve assembly of any one of the preceding claims, wherein at least one of the funnel or the enclosure is in fluid communication with a source of fluid of the medical device, and a fluid received at the funnel or the enclosure from the source of fluid is configured to move at least one of the first plate or the second plate relative to the other.

12. The valve assembly of claim 11, wherein the funnel is configured to guide the fluid toward the agent received therein to agitate the agent within the funnel.

13. The valve assembly of claim 12, wherein the funnel is in fluid communication with a delivery conduit (34, 36) of the medical device, and configured to guide a mixture of the fluid and the agent received from the funnel toward the delivery conduit.

14. The valve assembly of any one of the preceding claims, further including an actuator (30) coupled to at least one of the enclosure or the funnel, wherein the actuator is configured to move at least one of the first plate or the second plate relative to the other.

## Patentansprüche

1. Ventilanordnung (200A-200E) für eine medizinische Vorrichtung (10), aufweisend:
ein Gehäuse (210) zum Speichern eines Mittels, wobei das Gehäuse ein erstes Ende (214) aufweist;
einen Trichter (220), der stromabwärts des Gehäuses angeordnet ist, zum Aufnehmen des Mittels aus dem Gehäuse, wobei der Trichter ein zweites Ende (224) aufweist, das benachbart zu dem ersten Ende angeordnet ist;
eine erste Platte (216), die mit dem Gehäuse an dem ersten Ende gekoppelt ist, wobei die erste Platte zumindest eine Öffnung (218) aufweist; und
eine zweite Platte (226), die mit dem Trichter an dem zweiten Ende gekoppelt ist, wobei die zweite Platte zumindest eine Öffnung (228) aufweist, die in Fluidverbindung mit der zumindest einen Öffnung der ersten Platte steht;
wobei die erste Platte und die zweite Platte gegeneinander liegend angeordnet sind, und eine Außenfläche von zumindest einer aus der ersten Platte oder der zweiten Platte ein oder mehrere strukturierte Oberflächenelemente (219A, 219C, 229A, 229B, 229C) aufweist, die dazu konfiguriert sind, das in dem Gehäuse gespeicherte Mittel in Antwort darauf zu verrücken, dass sich zumindest eine aus der ersten Platte oder der zweiten Platte relativ zueinander bewegen;
wobei das eine oder die mehreren strukturierten Oberflächenelemente dazu konfiguriert sind, das Gehäuse in Vibration zu versetzen, in Antwort darauf, dass sich zumindest eine aus der ersten Platte oder der zweiten Platte relativ zueinander bewegen, um das in dem Gehäuse gespeicherte Mittel zu verrücken.

2. Ventilanordnung nach Anspruch 1, wobei das eine oder die mehreren strukturierten Oberflächenelemente einen Grat, einen Vorsprung, einen Ansatz, eine Unebenheit, eine Rippe, einen Zahn, eine Prallplatte, eine Vertiefung, eine Ausnehmung oder einen Hohlraum umfassen.

3. Ventilanordnung nach irgendeinem der vorhergehenden Ansprüche, wobei die erste Platte dazu konfiguriert ist, sich relativ zur zweiten Platte zu drehen oder zu verschieben.

4. Ventilanordnung nach irgendeinem der vorhergehenden Ansprüche, wobei das eine oder die mehreren strukturierten Oberflächenelemente dazu konfiguriert sind, das Mittel in dem Gehäuse aufzuschütteln und das aufgeschüttelte Mittel aus dem Gehäuse und in den Trichter abzugeben, in Antwort darauf, dass sich zumindest eine aus der ersten Platte oder der zweiten Platte relativ zueinander bewegen.

5. Ventilanordnung nach irgendeinem der vorhergehenden Ansprüche, wobei die Außenfläche von jeder aus der ersten Platte und der zweiten Platte ein oder mehrere strukturierte Oberflächenelemente aufweist.

6. Ventilanordnung nach Anspruch 5, wobei das eine oder die mehreren strukturierten Oberflächenelemente, die auf der Außenfläche der ersten Platte angeordnet sind, dazu konfiguriert sind, mit dem einen oder den mehreren strukturierten Oberflächenelementen, die auf der Außenfläche der zweiten Platte angeordnet sind, in Eingriff zu treten, um so das in dem Gehäuse gespeicherte Mittel zu verrücken.

7. Ventilanordnung nach Anspruch 6, wobei zumindest eine aus der ersten Platte oder der zweiten Platte dazu konfiguriert ist, sich in einer Längsrichtung relativ zueinander zu verschieben, wenn das eine oder die mehreren strukturierten Oberflächenelemente der ersten Platte mit dem einen oder den mehreren strukturierten Oberflächenelementen der zweiten Platte in Eingriff treten.

8. Ventilanordnung nach irgendeinem der vorhergehenden Ansprüche, wobei jede aus der ersten Platte und der zweiten Platte dazu konfiguriert ist, sich relativ zueinander zu bewegen, um das in dem Gehäuse gespeicherte Mittel zur Abgabe in den Trichter zu verrücken.

9. Ventilanordnung nach irgendeinem der vorhergehenden Ansprüche, wobei das eine oder die mehreren strukturierten Oberflächenelemente, die auf der Außenfläche von zumindest einer aus der ersten Platte oder der zweiten Platte angeordnet sind, dazu konfiguriert sind, sich in die zumindest eine Öffnung auf der ersten Platte oder der zweiten Platte zu erstrecken, die gegenüber dem einen oder den mehreren strukturierten Oberflächenelementen angeordnet ist.

10. Ventilanordnung nach irgendeinem der vorhergehenden Ansprüche, wobei die erste Platte und die zweite Platte porös sind, sodass jede aus der ersten Platte und der zweiten Platte eine Mehrzahl von Öffnungen aufweist, die in Fluidverbindung miteinander stehen.

11. Ventilanordnung nach irgendeinem der vorhergehenden Ansprüche, wobei zumindest einer aus dem Trichter oder dem Gehäuse in Fluidverbindung mit einer Fluidquelle der medizinischen Vorrichtung steht, und ein von der Fluidquelle an dem Trichter oder dem Gehäuse aufgenommenes Fluid dazu konfiguriert ist, zumindest eine aus der ersten Platte oder der zweiten Platte relativ zur anderen zu bewegen.

12. Ventilanordnung nach Anspruch 11, wobei der Trichter zum Leiten des Fluids zu dem darin aufgenommenen Mittel hin konfiguriert ist, um das Mittel innerhalb des Trichters aufzuschütteln.

13. Ventilanordnung nach Anspruch 12, wobei der Trichter in Fluidverbindung mit einer Abgabeleitung (34, 36) der medizinischen Vorrichtung steht, und zum Leiten eines Gemischs aus dem Fluid und dem vom Trichter aufgenommenen Mittel zur Abgabeleitung hin konfiguriert ist.

14. Ventilanordnung nach irgendeinem der vorhergehenden Ansprüche, ferner aufweisend einen Aktuator (30), der mit zumindest einem aus dem Gehäuse oder dem Trichter gekoppelt ist, wobei der Aktuator zum Bewegen zumindest einer aus der ersten Platte oder der zweiten Platte relativ zur anderen konfiguriert ist.

## Revendications

1. Ensemble de vanne (200A-200E) pour un dispositif médical (10), comprenant:
une enceinte (210) pour stocker un agent, l'enceinte incluant une première extrémité (214);
un entonnoir (220) positionné en aval de l'enceinte pour recevoir l'agent provenant de l'enceinte, l'entonnoir incluant une deuxième extrémité (224) positionnée de manière adjacente à la première extrémité;
une première plaque (216) couplée à l'enceinte à la première extrémité, la première plaque incluant au moins une ouverture (218); et
une deuxième plaque (226) couplée à l'entonnoir à la deuxième extrémité, la deuxième plaque incluant au moins une ouverture (228) qui est en communication fluidique avec la au moins une ouverture de la première plaque;
dans lequel la première plaque et la deuxième plaque sont positionnées l'une contre l'autre, et une surface extérieure d'au moins l'une de la première plaque ou de la deuxième plaque inclut un ou plusieurs éléments de surface texturés (219A, 219C, 229A, 229B, 229C) configurés pour déplacer l'agent stocké dans l'enceinte en réponse à ce qu'au moins l'une de la première plaque ou de la deuxième plaque se déplace par rapport à l'autre ;
dans lequel les un ou plusieurs éléments de surface texturés sont configurés pour faire vibrer l'enceinte, en réponse à ce qu'au moins l'une de la première plaque ou de la deuxième plaque se déplace par rapport à l'autre, pour déplacer l'agent stocké dans l'enceinte.

2. Ensemble de vanne selon la revendication 1, dans lequel les un ou plusieurs éléments de surface texturés incluent une arête, une protubérance, une saillie, une bosse, une nervure, une dent, un déflecteur, une alvéole, un évidement ou une cavité.

3. Ensemble de vanne selon l'une quelconque des revendications précédentes, dans lequel la première plaque est configurée pour tourner ou se déplacer par rapport à la deuxième plaque.

4. Ensemble de vanne selon l'une quelconque des revendications précédentes, dans lequel les un ou plusieurs éléments de surface texturés sont configurés pour agiter l'agent dans l'enceinte et libérer l'agent agité de l'enceinte et dans l'entonnoir en réponse à ce qu'au moins l'une de la première plaque ou de la deuxième plaque se déplace par rapport à l'autre.

5. Ensemble de vanne selon l'une quelconque des revendications précédentes, dans lequel la surface extérieure de chacune de la première plaque et de la deuxième plaque inclut un ou plusieurs éléments de surface texturés.

6. Ensemble de vanne selon la revendication 5, dans lequel les un ou plusieurs éléments de surface texturés positionnés sur la surface extérieure de la première plaque sont configurés pour entrer en contact avec les un ou plusieurs éléments de surface texturés positionnés sur la surface extérieure de la deuxième plaque, déplaçant ainsi l'agent stocké dans l'enceinte.

7. Ensemble de vanne selon la revendication 6, dans lequel au moins l'une de la première plaque ou de la deuxième plaque est configurée pour se déplacer dans une direction longitudinale par rapport à l'autre lorsque les un ou plusieurs éléments de surface texturés de la première plaque entrent en contact avec les un ou plusieurs éléments de surface texturés de la deuxième plaque.

8. Ensemble de vanne selon l'une quelconque des revendications précédentes, dans lequel chacune de la première plaque et de la deuxième plaque sont configurées pour se déplacer par rapport à l'autre pour déplacer l'agent stocké dans l'enceinte et pour le libérer dans l'entonnoir.

9. Ensemble de vanne selon l'une quelconque des revendications précédentes, dans lequel les un ou plusieurs éléments de surface texturés positionnés sur la surface extérieure d'au moins l'une de la première plaque ou de la deuxième plaque sont configurés pour s'étendre dans la au moins une ouverture sur la première plaque ou la deuxième plaque qui est positionnée de manière opposée aux un ou plusieurs éléments de surface texturés.

10. Ensemble de vanne selon l'une quelconque des revendications précédentes, dans lequel la première plaque et la deuxième plaque sont poreuses, de sorte que chacune de la première plaque et de la deuxième plaque inclut une pluralité d'ouvertures qui sont en communication fluidique entre elles.

11. Ensemble de vanne selon l'une quelconque des revendications précédentes, dans lequel au moins l'un de l'entonnoir ou de l'enceinte est en communication fluidique avec une source de fluide du dispositif médical, et un fluide reçu au niveau de l'entonnoir ou de l'enceinte depuis la source de fluide est configuré pour déplacer au moins l'une de la première plaque ou de la deuxième plaque par rapport à l'autre.

12. Ensemble de vanne selon la revendication 11, dans lequel l'entonnoir est configuré pour guider le fluide vers l'agent reçu à l'intérieur pour agiter l'agent dans l'entonnoir.

13. Ensemble de vanne selon la revendication 12, dans lequel l'entonnoir est en communication fluidique avec un conduit de délivrance (34, 36) du dispositif médical, et configuré pour guider un mélange du fluide et de l'agent reçu de l'entonnoir vers le conduit de délivrance.

14. Ensemble de vanne selon l'une quelconque des revendications précédentes, incluant en outre un actionneur (30) couplé à au moins l'un de l'enceinte ou de l'entonnoir, dans lequel l'actionneur est configuré pour déplacer au moins l'une de la première plaque ou de la deuxième plaque par rapport à l'autre.
